(19)

Europäisches
Patentamt
European
Patent Office
Office européen
des brevets

(11) **EP 4 350 000 A1**

(12) **EUROPEAN PATENT APPLICATION**
published in accordance with Art. 153(4) EPC

(43) Date of publication:
**10.04.2024 Bulletin 2024/15**

(21) Application number: **22811183.7**

(22) Date of filing: **16.05.2022**

(51) International Patent Classification (IPC):
***C12Q 1/04*** (2006.01)  ***C12N 1/20*** (2006.01)
***G01N 33/48*** (2006.01)

(52) Cooperative Patent Classification (CPC):
**C12N 1/20; C12Q 1/04; G01N 33/48**

(86) International application number:
**PCT/JP2022/020322**

(87) International publication number:
**WO 2022/249910 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.05.2021 JP 2021088206**

(71) Applicant: **Kabushiki Kaisha Yakult Honsha
Tokyo, 105-8660 (JP)**

(72) Inventors:
• **KUBOTA Hiroyuki
Tokyo 105-8660 (JP)**
• **SERATA Masaki
Tokyo 105-8660 (JP)**
• **OKUMURA Takekazu
Tokyo 105-8660 (JP)**

(74) Representative: **Wächtershäuser & Hartz
Patentanwaltspartnerschaft mbB
Weinstraße 8
80333 München (DE)**

(54) **METHOD FOR DETECTING LIVE LACTIC ACID BACTERIA WITH CARBOHYDRATE
METABOLISM ACTIVITY**

(57)     In order to provide a method capable of rapidly measuring live lactic acid bacteria in a lactic acid bacteria-containing beverage or food, a method for detecting live lactic acid bacteria with carbohydrate metabolism activity contained in a lactic acid bacteria-containing beverage or food is characterized by including the following steps (a) to (c): (a) a step of adding a reagent that exhibits fluorescence when degraded by an esterase and a reagent for determining cell membrane damage to a lactic acid bacteria-containing beverage or food; (b) a step of adjusting a hexose having an aldehyde group contained in the lactic acid bacteria-containing beverage or food to 0.01 mM or more; and (c) a step of detecting lactic acid bacteria that excrete a fluorescent substance and have no cell membrane damage from the lactic acid bacteria-containing beverage or food.

Fig.1

EP 4 350 000 A1

**Description**

Technical Field

The present invention relates to a method for detecting live lactic acid bacteria with carbohydrate metabolism activity contained in a lactic acid bacteria-containing beverage or food.

Background Art

Lactic acid bacteria bring about various physiological effects such as an intestinal regulation action, and therefore are widely consumed as yogurt or lactic acid bacteria beverages. In order to obtain these physiological effects, it is very important to ensure a predetermined live cell count and maintain the live cell count in a product. In addition, it is important to control the live cell count also for controlling the effect of an acid produced by lactic acid bacteria on the flavor of a product, or the like, and therefore, there is a need for a method for rapidly measuring the live cell count of lactic acid bacteria.

As a method for measuring the live cell count of lactic acid bacteria, there is a method in which an analyte containing lactic acid bacteria is applied to an agar medium in which lactic acid bacteria can grow, and colonies obtained by culturing are counted. However, it takes more than ten hours to several days until the measurement can be carried out, and therefore, the method has a problem that a time lag occurs until the result of the live cell count is obtained (PTL 1).

In addition, a method for detecting active bacteria using esterase activity and cell membrane permeability as indices employing a double staining method with a reagent that exhibits fluorescence when degraded by an esterase (carboxyfluorescein diacetate: cFDA) and a reagent for examining cell membrane damage (propidium iodide: PI) is also known (in this method, a bacterium that is stained with cF to be generated by degradation of cFDA (cF$^+$) and has no membrane damage (PI-) is regarded as an active bacterium), however, in an environment such as in a low-temperature fermented milk, the cell membrane or esterase activity may be maintained even in dead bacteria, and live bacteria could not be measured by this method in some cases (NPL 1) .

In addition, many measurement methods using a reagent capable of staining only live or dead bacteria have been reported, but there is a need for a simpler and more highly sensitive method.

Citation List

Patent Literature

PTL 1: JPH04-218392A

Non Patent Literature

NPL 1: Lahtinen SJ, Ouwehand AC, Reinikainen JP, Korpela JM, Sandholm J, Salminen SJ. 2006. Intrinsic properties of so-called dormant probiotic bacteria, determined by flow cytometric viability assays. Appl Environ Microbiol 72: 5132-4

Summary of Invention

Technical Problem

A problem to be solved by the invention is to provide a method capable of rapidly measuring live lactic acid bacteria in a lactic acid bacteria-containing beverage or food.

Solution to Problem

As a result of intensive studies for solving the above problem, the present inventors found that live lactic acid bacteria with carbohydrate metabolism activity can be rapidly detected by adding a reagent that exhibits fluorescence when degraded by an esterase and a reagent for determining cell membrane damage to a lactic acid bacteria-containing beverage or food, and thereafter allowing a hexose having an aldehyde group to be present in a specific amount or more, and then, detecting lactic acid bacteria that excrete a fluorescent substance outside the bacterial cells and have no cell membrane damage, and completed the invention. In addition, they found that lactic acid bacteria with high carbohydrate metabolism activity can be selected by utilizing the above finding, and further, the acid resistance of lactic acid bacteria and the viability of lactic acid bacteria after low-temperature storage can be predicted by utilizing this finding, and completed the invention.

That is, the invention is a method for detecting live lactic acid bacteria with carbohydrate metabolism activity contained in a lactic acid bacteria-containing beverage or food, characterized by including the following steps (a) to (c):

(a) a step of adding a reagent that exhibits fluorescence when degraded by an esterase and a reagent for determining cell membrane damage to a lactic acid bacteria-containing beverage or food;
(b) a step of adjusting a hexose having an aldehyde group contained in the lactic acid bacteria-containing beverage or food to 0.01 mM or more; and
(c) a step of detecting lactic acid bacteria that excrete a fluorescent substance and have no cell membrane damage from the lactic acid bacteria-containing beverage or food.

Further, the invention is a method for selectively detecting live lactic acid bacteria with high carbohydrate metabolism activity, characterized by including the following steps (d) to (f):

(d) a step of adding a reagent that exhibits fluorescence when degraded by an esterase and a reagent for determining cell membrane damage to an analyte containing lactic acid bacteria;
(e) a step of adjusting a hexose having an aldehyde group in the analyte to 0.01 mM or more; and
(f) a step of detecting lactic acid bacteria that excrete a fluorescent substance and have no cell membrane damage from the analyte within 10 minutes after the step (e).

In addition, the invention is a method for predicting acid resistance of lactic acid bacteria contained in an analyte or a method for predicting viability after low-temperature storage of lactic acid bacteria contained in an analyte, characterized by using the above-mentioned selective detection method.

Advantageous Effects of Invention

According to the invention, it is possible to rapidly (in real time during culturing) detect live lactic acid bacteria with carbohydrate metabolism activity, which bring about various physiological effects and affect the acidity of a fermented product.

In addition, according to the invention, it is possible to selectively detect live lactic acid bacteria with high carbohydrate metabolism activity, which bring about various physiological effects and affect the acidity of a fermented product.

Further, according to the invention, it is possible to predict the acid resistance of lactic acid bacteria selectively detected above or predict the viability thereof after low-temperature storage.

Brief Description of Drawings

[FIG. 1] FIG. 1 is a view showing the results of flow cytometry after adding reagents in a lactic acid bacteria-containing beverage or food stored at about 4°C for 5 days after production in Example 1 (in the drawing, A denotes inactive bacteria (cF-, PI+), B denotes damaged bacteria (cF±, PI±), C denotes conventional active bacteria (cF+, PI-), and D denotes lactic acid bacteria with carbohydrate metabolism activity detected by the invention (cF-, PI-)).

[FIG. 2] FIG. 2 is a view showing the results of flow cytometry after adding reagents in a lactic acid bacteria-containing beverage or food stored at about 4°C for 218 days after production in Example 2 (in the drawing, A denotes inactive bacteria (cF-, PI+), B denotes damaged bacteria (cF±, PI±), C denotes conventional active bacteria (cF+, PI-), and D denotes lactic acid bacteria with carbohydrate metabolism activity detected by the invention (cF-, PI-)).

[FIG. 3] FIG. 3 is a view showing a correlation between the cell count of LcS exhibiting carbohydrate metabolism activity or the like and a colony-forming ability in Example 3.

[FIG. 4] FIG. 4 is a view showing the time course of cF accumulation and cF excretion of LcS in Reference Example 1.

[FIG. 5] FIG. 5 is a view showing changes in the percentage of LcS with cF excretion activity after adding glucose in Reference Example 4.

[FIG. 6] FIG. 6 shows the FL2-SS histograms of 2-fold serially diluted samples of LcS in Reference Example 5.

[FIG. 7] FIG. 7 is a view showing the measured value and the theoretical value of the live cell count by flow cytometry in 2-fold serially diluted bacterial solutions in Reference Example 5.

[FIG. 8] FIG. 8 is a view showing the percentage of beads and the live cell count by flow cytometry in an LcS undiluted solution in Reference Example 5.

[FIG. 9] FIG. 9 is a view showing the results of FCM analysis of a lactic acid bacteria-containing beverage or food in Example 6 (● in the drawing indicates a measurement point).

[FIG. 10] FIG. 10 is a view obtained by plotting the excretion score and the percentage of LcS with excretion activity of a lactic acid bacteria-containing beverage or food in Example 6.

Description of Embodiments

The method for detecting live lactic acid bacteria with carbohydrate metabolism activity contained in a lactic acid bacteria-containing beverage or food of the invention (hereinafter referred to as "the detection method of the invention") includes the following steps (a) to (c), and preferably, the following steps (a) to (c) are performed in this order.

(a) a step of adding a reagent that exhibits fluorescence when degraded by an esterase and a reagent for determining cell membrane damage to a lactic acid bacteria-containing beverage or food
(b) a step of adjusting a hexose having an aldehyde group contained in the lactic acid bacteria-containing beverage or food to 0.01 mM or more
(c) a step of detecting lactic acid bacteria that excrete a fluorescent substance and have no cell membrane damage from the lactic acid bacteria-containing beverage or food

The lactic acid bacteria-containing beverage or food used in the step (a) is not particularly limited as long as it is a beverage or food containing lactic acid bacteria in the form of live bacteria, but is, for example, one containing a soy milk such as a fermented soy milk, one containing a fruit juice such as a fermented fruit juice, or a vegetable juice, one containing a milk component such as a dairy product lactic acid bacteria beverage, a lactic acid bacteria beverage, kefir, yogurt, or a fermented milk, or the like. Among these lactic acid bacteria-containing beverages or foods, one containing a milk component is preferred. Further, examples of the form include a hard type, a soft type, a plain type, a sweet type, a fruit type, a drink type, and a frozen type. In order to mix the reagents uniformly, it is desirable to remove solids in advance by filtration or the like when solids are contained. In addition, in the case of a hard type, it is preferred to perform dilution using Dulbecco's PBS (hereinafter sometimes referred to as DPBS) or the like to convert it into a liquid form.

In addition, the lactic acid bacteria contained in the lactic acid bacteria-containing beverage or food are not particularly limited as long as they have metabolism activity for a hexose having an aldehyde group. As such lactic acid bacteria, for example, lactic acid bacteria belonging to the Lactobacillus family and lactic acid bacteria belonging to the Streptococcus family are exemplified, and more specifically, lactic acid bacteria belonging to the genus Streptococcus, lactic acid bacteria belonging to the genus Lactococcus such as Lactococcus lactis, lactic acid bacteria belonging to the genus Lacticaseibacillus such as Lacticaseibacillus paracasei and Lacticaseibacillus casei, lactic acid bacteria belonging to the genus Lactobacillus such as Lactobacillus acidophilus, Lactobacillus gasseri, and Lactobacillus helveticus, lactic acid bacteria belonging to the genus Lactiplantibacillus such as Lactiplantibacillus plantarum, lactic acid bacteria belonging to the genus Levilactobacillus such as Levilactobacillus brevis, lactic acid bacteria belonging to lactic acid bacteria belonging to the genus Ligilactobacillus such as Ligilactobacillus salivarius, and the like are exemplified. Among these lactic acid bacteria, preferred families are Lactobacillus family and Streptococcus family, and preferred genera are genus Lacticaseibacillus, genus Lactobacillus, genus Lactiplantibacillus, genus Levilactobacillus, genus Ligilactobacillus, and genus Lactococcus, and preferred bacterial strains are Lacticaseibacillus paracasei, Lacticaseibacillus casei, Lactobacillus acidophilus, Lactobacillus gasseri, Lactobacillus helveticus, Lactiplantibacillus plantarum, Levilactobacillus brevis, Ligilactobacillus salivarius, and Lactococcus lactis. As the lactic acid bacteria, one strain or two or more strains may be contained in the lactic acid bacteria-containing beverage or food. Further, the lactic acid bacteria are preferably diluted so that the cell count of lactic acid bacteria contained in the lactic acid bacteria-containing beverage or food is to $10^5$ to $10^6$ cells/mL. For the dilution, sterilized DPBS or the like can be used.

Whether the lactic acid bacteria have metabolism activity for a hexose having an aldehyde group can be confirmed by a kit for testing carbohydrate metabolism, for example, an API 50cH test using a hexose having an aldehyde group described later, or the like. This confirmation is preferably performed in advance.

Examples of the reagent that exhibits fluorescence when degraded by an esterase used in the step (a) include carboxyfluorescein diacetate (cFDA), BCECF acetoxymethyl ester (BCECF-AM, chemical name: 3'-O-Acetyl-2',7'-bis(carboxyethyl)-4 or 5-carboxyfluorescein, diacetoxymethyl ester), and calcein acetoxymethyl ester (Calcein-AM, chemical name: 3',6'-Di(O-acetyl)-4',5'-bis[N,N-bis(carboxymethyl)aminomethyl]fluorescein, tetraacetoxymethyl ester).

Further, examples of the reagent for determining cell membrane damage used in the step (a) include propidium iodide and Sytox (Thermo Fisher Scientific K.K., Sumitomo Fudosan Mita Twin Bldg, East wing. 4-2-8, Shibaura, Minato-Ku, Tokyo), and particularly Sytox orange, Sytox green, Sytox red, and Sytox blue are exemplified.

In the step (a), each reagent may be added to the lactic acid bacteria-containing beverage or food at 10 μM to 120 μM, preferably 20 μM to 100 μM or so. The temperature and pH of the lactic acid bacteria-containing beverage or food when these reagents are added are not particularly limited, but for example, it is preferred to set the temperature to 15 to 40°C and the pH to 4 to 10.

Examples of the hexose having an aldehyde group used in the step (b) include glucose, galactose, and mannose. Among these hexoses having an aldehyde group, one type or two or more types can be used.

In the step (b), a method for adjusting the hexose having an aldehyde group contained in the lactic acid bacteria-containing beverage or food to 0.01 mM or more, preferably 0.1 mM or more is not particularly limited, but when the lactic acid bacteria-containing beverage or food contains the hexose having an aldehyde group at 0.01 mM or more, no particular action is required, and when the lactic acid bacteria-containing beverage or food does not contain the hexose having an aldehyde group at 0.01 mM or more, the hexose having an aldehyde group may be added to adjust the amount to the above-mentioned value or more.

Before the step (a), a saccharide originally contained in the lactic acid bacteria-containing beverage or food may be removed. A method for removing the saccharide from the lactic acid bacteria-containing beverage or food is not particularly limited, but for example, a method in which lactic acid bacteria are collected from the lactic acid bacteria-containing beverage or food by centrifugation or the like and suspended in a culture medium that does not contain a hexose having an aldehyde group is exemplified. As a liquid that can be used for suspension, DPBS or the like is exemplified, and it is preferably used after performing a sterilization treatment. When the treatment of removing the carbohydrate is performed, it is, of course, necessary to newly perform adjustment so that the hexose having an aldehyde group is contained in the lactic acid bacteria-containing beverage or food at 0.01 mM or more in the step (b). By removing the carbohydrate in advance before the step (a), it is possible to control the time for the lactic acid bacteria to contact with a sugar from the addition of the reagents in the step (a) to the step (c), and the conditions can be equalized when performing measurements for multiple analytes or periodic measurements.

In the step (c), a method for detecting lactic acid bacteria that excrete a fluorescent substance and have no cell membrane damage from the lactic acid bacteria-containing beverage or food is not particularly limited, but the detection can be performed by flow cytometry using a double staining method. In this method, lactic acid bacteria that excrete a fluorescent substance are detected as cF$^-$, and lactic acid bacteria that have no cell membrane damage are detected as PI$^-$. A flow cytometer to be used is not particularly limited, and conditions may be appropriately set according to an analyte or a reagent to be used.

In a conventional double staining method, cF, which is a degradation product of cFDA by an intracellular esterase, accumulates in the cell membrane and fluorescently stained bacterial cells (cF$^+$) are detected as active bacteria, however, in the detection method of the invention, conversely, bacterial cells that excrete cF (cF$^-$) are detected as live bacteria. This makes it possible to also measure lactic acid bacteria in an analyte, for which live bacteria could not be accurately measured so far by a double staining method, such as a lactic acid bacteria-containing beverage or food stored at a low temperature. As will be described later, the excretion of cF occurs in the presence of a hexose having an aldehyde group, and therefore, it is considered that cF is excreted by metabolizing such a sugar. Accordingly, in the detection method of the invention, a bacterial cell (cF$^-$) that performs cF excretion is a bacterial cell that has carbohydrate metabolism activity for a hexose having an aldehyde group. In the present specification, exhibiting cF excretion in the presence of a hexose having an aldehyde group is sometimes referred to as "cF excretion activity", "excretion activity", or "carbohydrate metabolism activity".

After the step (b), the step (c) is preferably performed within 60 minutes, more preferably within 15 minutes or more and 30 minutes or less. If the time until detection is too long, accumulation of the fluorescent substance may be observed even though the bacteria are alive, resulting in erroneous determination.

In the detection method of the invention, the absolute number of live lactic acid bacteria can be measured together with the detection of live lactic acid bacteria by adding beads for concentration measurement to the lactic acid bacteria-containing beverage or food. The concentration of the beads for concentration measurement is not particularly limited, but is preferably set to 5 to 70% of the total particles contained in the lactic acid bacteria-containing beverage or food when the beads for concentration measurement are added to the lactic acid bacteria-containing beverage or food. The addition of the beads for concentration measurement to the lactic acid bacteria-containing beverage or food is preferably performed after the step (a) or the step (b). For example, the amount of the beads to be added can be determined from the cell count of lactic acid bacteria obtained from a turbidity or an absorbance and the total amount of the beads, and when the cell count of lactic acid bacteria contained in the lactic acid bacteria-containing beverage or food is set to $10^5$ cells/mL, an equal volume of the beads at a concentration of about $5.3 \times 10^3$ to $2.3 \times 10^5$ /mL are added. After adding the beads, it is preferred to perform stirring in advance so that the beads are uniformly dispersed in the lactic acid bacteria-containing beverage or food.

In a preferred embodiment of the detection method of the invention, a method for detecting live bacteria in a fermented milk containing Lacticaseibacillus paracasei YIT 9029 strain (former name: Lactobacillus casei YIT 9029, hereinafter sometimes referred to as "LcS") as the lactic acid bacteria-containing beverage or food is exemplified. According to this, live LcS bacteria having carbohydrate metabolism activity can be rapidly detected (in real time during culturing).

The above-mentioned Lacticaseibacillus paracasei YIT 9029 strain (LcS) has been internationally deposited in International Patent Organism Depositary, National Institute of Technology and Evaluation (#120, 2-5-8 Kazusakamatari, Kisarazu-shi, Chiba-ken, 292-0818, Japan), which is an international depositary authority as stipulated by the Budget Treaty, as Lactobacillus casei YIT 9029 (FERM BP-1366, date of deposit: May 1, 1981). LcS was previously classified as Lactobacillus casei, but in recent years, the genus Lactobacillus was reclassified, and the strain was classified as Lacticaseibacillus paracasei (Zheng et al., A taxonomic note on the genus Lactobacillus: Description of 23 novel genera, emended description of the genus Lactobacillus Beijerinck 1901, and union of Lactobacillaceae and Leuconostocaceae. Int. J. Syst. Evol. Microbiol. 2020 Apr; 70(4): 2782-2858 DOI 10.1099/ijsem.0.004107). In this specification, the classification of lactic acid bacteria is indicated by the new classification classified based on the above literature.

By using the detection method of the invention, live lactic acid bacteria with high carbohydrate metabolism activity can be selectively detected. Specifically, the method for selectively detecting live lactic acid bacteria with high metabolism activity for a hexose having an aldehyde group of the invention (hereinafter referred to as the "selective detection method of the invention") includes the following steps (d) to (f), and preferably, the steps are performed in this order.

(d) a step of adding a reagent that exhibits fluorescence when degraded by an esterase and a reagent for determining cell membrane damage to an analyte containing lactic acid bacteria
(e) a step of adjusting a hexose having an aldehyde group in the analyte to 0.01 mM or more
(f) a step of detecting lactic acid bacteria that excrete a fluorescent substance and have no cell membrane damage from the analyte within 10 minutes after the step (e)

The analyte containing lactic acid bacteria used in the step (d) is not particularly limited as long as it contains lactic acid bacteria, and in addition to the lactic acid bacteria-containing beverage or food used in the detection method of the invention, a culture solution obtained by culturing lactic acid bacteria in a culture medium such as MRS medium, or the like is exemplified. Among these analytes containing lactic acid bacteria, a lactic acid bacteria-containing beverage or food is preferred, and a lactic acid bacteria-containing beverage or food containing a milk component is particularly preferred.

In the selective detection method of the invention, the analyte containing lactic acid bacteria used in the step (d) should be one from which a saccharide has been removed if the analyte contains a saccharide. A method for removing a saccharide having an aldehyde group from the analyte is the same as that for the detection method of the invention. In order to select bacteria with high carbohydrate metabolism activity, it is necessary to strictly measure the excretion time of a fluorescent reagent. This is because the excretion of a fluorescent substance starts when adding the reagent that exhibits fluorescence when degraded by an esterase if the analyte contains a saccharide, which makes it difficult to control the timing of performing selective detection of bacteria with high carbohydrate metabolism activity. The method for removing a saccharide from the analyte is not particularly limited, but for example, a method in which lactic acid bacteria are collected from the analyte by centrifugation or the like and suspended in a culture medium that does not contain a hexose having an aldehyde group is exemplified. Further, in the case of a lactic acid bacteria-containing beverage or food containing a milk component, a method in which a milk protein is degraded by a protease treatment, and thereafter, lactic acid bacteria are collected from the analyte by centrifugation or the like and suspended in a culture medium that does not contain a hexose having an aldehyde group, or the like is exemplified. As a liquid that can be used for suspension, DPBS or the like is exemplified, and it is preferably used after performing a sterilization treatment. When the analyte does not contain a saccharide, it is not necessary to perform an operation of removing a saccharide as described above.

The reagent that exhibits fluorescence when degraded by an esterase used in the step (d) is the same as in the step (a).

In addition, the reagent for determining cell membrane damage used in the step (d) is the same as in the step (a).

The addition amount of each reagent in the step (d) is the same as in the step (a) . Further, when adding these reagents, it is preferred to set the temperature of the analyte to 15 to 40°C and the pH of the analyte to 4 to 10, which is also the same as in the step (a).

In the step (e), the hexose having an aldehyde group to be used is the same as in the step (b).

In the step (e), a method for adjusting the hexose having an aldehyde group contained in the lactic acid bacteria-containing analyte to 0.01 mM or more, preferably 0.1 M or more is the same as in the step (b).

In the step (f), a method for detecting lactic acid bacteria that excrete a fluorescent substance and have no cell membrane damage from the analyte is the same as in the step (c). In the step (f), the measurement may be performed within 10 minutes after the step (e). Note that the excretion rate of the fluorescent substance or the like from lactic acid bacteria, or the like may increase depending on the composition of the culture medium, the strain of bacteria, other conditions, etc., and therefore, the time until measurement may be appropriately adjusted accordingly.

Specifically, in the step (f), the amount of lactic acid bacteria that excrete a fluorescent substance and have no cell membrane damage detected at a certain one point during the elapsed time is compared, and an analyte in which the amount is larger can be selected as an analyte containing a large amount of live lactic acid bacteria with high metabolism activity for a hexose having an aldehyde group of the invention.

In the selective detection method of the invention, in the step (f), lactic acid bacteria that excrete a fluorescent substance and have no cell membrane damage are detected from the analyte within 10 minutes, but when the excretion of a fluorescent substance from the analyte is fast, such as in the case were the carbohydrate metabolism activity of the analyte is very high or the like, it is preferred that in the step (f), the percentage of lactic acid bacteria that have no cell membrane damage and have completed the excretion of the fluorescent substance is plotted on a vertical axis and the elapsed time after adjusting the hexose having an aldehyde group to 0.01 mM or more is plotted on the horizontal axis, and the detection of lactic acid bacteria that have no excretion of the fluorescent substance nor cell membrane damage is performed based on an area value of the plotted curve. The area value can be calculated by performing trapezoidal approximation of areas between plots and adding up the areas.

Specifically, the detection can be performed by the following procedure.

After the step (e), the number of bacteria that have excreted the fluorescent substance is continuously measured until the excretion is completed.

The percentage of lactic acid bacteria that have completed the excretion of the fluorescent substance is plotted on the vertical axis, and the elapsed time after adjusting the hexose having an aldehyde group to 0.01 mM or more is plotted on the horizontal axis.

The area value of the plotted curve is calculated, preferably, the area value in a specific range during the elapsed time is calculated, and the area value or a value obtained by scoring the area value (excretion score) is compared, and an analyte in which the value is larger is selected as an analyte containing a large amount of live lactic acid bacteria with high metabolism activity for a hexose having an aldehyde group of the invention. A preferred range during the elapsed time for calculating the area value is a time from the start of excretion to the completion of excretion, however, when the excretion rate is extremely fast or slow, it may be a time from the early stage of excretion to the late stage of excretion. In addition, when comparing the area values or excretion scores of different analytes, as the ranges thereof, area values or excretion scores calculated within the same given range of time (for example, from 120 seconds to 570 seconds after adjusting the hexose having an aldehyde group in the analyte to 0.01 mM or more, or the like) are used. In addition, the excretion score is preferably a percentage calculated by dividing the area value of the plotted curve described above by the area of a rectangle assuming that the percentage of LcS that has completed the excretion in the corresponding time is always 100%.

Note that also in the selective detection method of the invention, in the same manner as in the detection method of the invention, the absolute number of live lactic acid bacteria with high carbohydrate metabolism activity can be measured together with the selection thereof by adding beads for concentration measurement to the analyte. The addition amount (concentration) of the beads for concentration measurement is not particularly limited, but is preferably set to 5 to 70% of the total particles when added to the analyte. The addition of the beads for concentration measurement is preferably performed after the step (d) or (e), and particularly preferably performed immediately before the step (f).

In addition, since it has been confirmed that an analyte in which the percentage of lactic acid bacteria with high carbohydrate metabolism activity is larger tends to exhibit higher acid resistance, the selective detection method of the invention can be used as a method for predicting the acid resistance of lactic acid bacteria contained in an analyte.

Further, it was found that CFU after low-temperature storage of the analyte was maintained at a high level according to the percentage of lactic acid bacteria with high carbohydrate metabolism activity. Therefore, the selective detection method of the invention can be used as a method for predicting the viability after low-temperature storage of lactic acid bacteria contained in an analyte, that is, as a method for predicting the viability after low-temperature storage of an analyte. The "low-temperature storage" as used herein refers to storage at a temperature of 10°C or lower. By performing the selective detection method of the invention immediately after culturing, it is possible to predict the viability after low-temperature storage of lactic acid bacteria contained in the analyte. In addition, by using the percentage of lactic acid bacteria with high carbohydrate metabolism activity as an index, the viability after low-temperature storage can be predicted without performing a storage test, and therefore, it is possible to efficiently study the production of an analyte with high viability after low-temperature storage.

Examples

Hereinafter, the invention will be described in detail with reference to Examples, however, the invention is by no means limited to these Examples.

Example 1

Detection of Live Lactic Acid Bacteria with Carbohydrate Metabolism Activity:

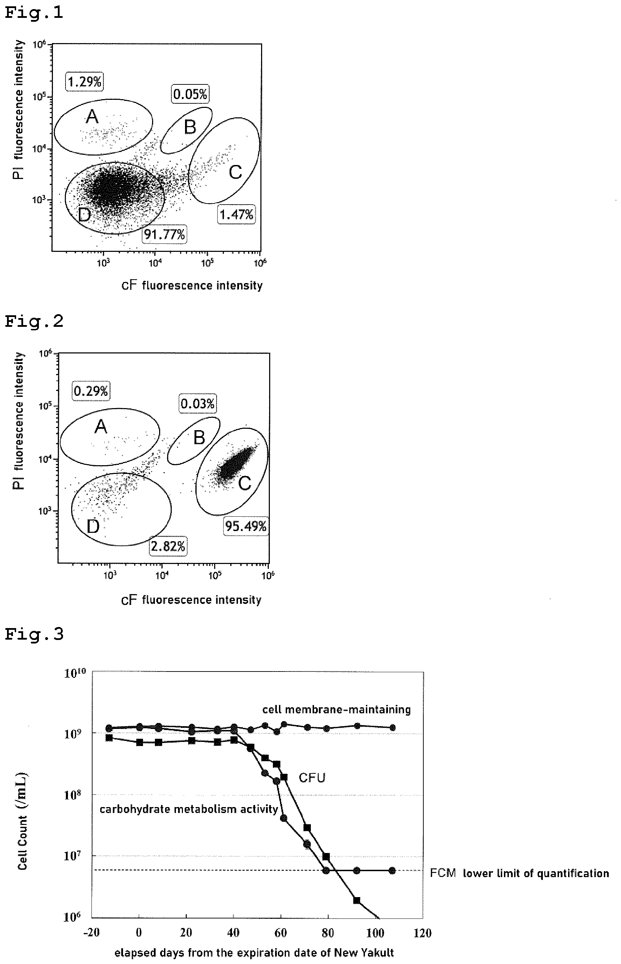

A beverage or food (New Yakult: manufactured by Yakult Honsha Co., Ltd.) containing Lacticaseibacillus paracasei YIT 9029 (LcS: FERM BP-1366) stored at 4°C for 5 days after production was used as an analyte. New Yakult was diluted $2 \times 10^3$ times with Dulbecco's PBS (DPBS: manufactured by Thermo Fisher Scientific, Inc., hereinafter simply referred to as DPBS) filtered through a 0.22 um filter, and 485 $\mu$L thereof was dispensed into a 1.5 mL tube. 5 $\mu$L of a DMSO solution containing 5 mM carboxyfluorescein diacetate (cFDA) was added thereto, and the resultant was left to stand at 30°C for 15 minutes, and then, 10 $\mu$L of a 1.5 mM propidium iodide (PI) solution was added thereto, and the resultant was left to stand at 30°C for 15 minutes. Thereafter, an analysis was performed by Gallios (Beckman Coulter). The number of particles to be analyzed was set to 10, 000. The results are shown in FIG. 1. Inactive bacteria (bacterial cells with no esterase activity but with membrane damage, hereinafter "cF⁻, PI⁺") are denoted by A, damaged bacteria (bacterial cells with weak esterase activity and some membrane damage, hereinafter "cF$^\pm$, PI$^\pm$") are denoted by B, conventional active bacteria (bacterial cells with esterase activity but no membrane damage, hereinafter "cF⁺, PI⁻") are denoted by C, and bacteria with carbohydrate metabolism activity detected by the invention (bacterial cells that have esterase activity and activity of excretion of generated cF but no membrane damage, hereinafter referred to as "cF⁻, PI⁻") are denoted by D.

As shown in FIG. 1, it was confirmed that the cell count of conventional active bacteria (those of cF⁺, PI⁻ denoted by C) was considerably small even though immediately after production. This result revealed that live bacteria cannot be accurately detected by the conventional method for detecting active bacteria using esterase activity and cell membrane permeability as indices employing a double staining method. In addition, as described above, since cF excretion occurs in the presence of a hexose having an aldehyde group, it is considered that cF is excreted by metabolizing such a sugar. Since most of LcS contained in New Yakult immediately after production is live bacteria, the results in FIG. 1 suggested that live lactic acid bacteria with carbohydrate metabolism activity can be accurately detected by detecting those of cF⁻, PI⁻ denoted by D.

Example 2

Detection of Live Lactic Acid Bacteria with Carbohydrate Metabolism Activity:

Flow cytometry was performed in the same manner as in Example 1 for New Yakult stored at about 4°C for 218 days after production (6 months past the expiration date). The results are shown in FIG. 2. A denotes inactive bacteria (cF⁻, PI⁺), B denotes damaged bacteria (cF$^\pm$, PI$^\pm$), C denotes conventional active bacteria (cF⁺, PI⁻), and D denotes lactic acid bacteria with carbohydrate metabolism activity detected by the invention (cF⁻, PI⁻).

It is known that in a lactic acid bacteria-containing beverage or food after long-term storage, most of the lactic acid bacteria contained therein are dead bacteria. It was clear that if those of cF⁺, PI⁻ are considered as active bacteria, the cell count is considerably large even after long-term storage, and live bacteria cannot be accurately detected. On the other hand, it was suggested that by using cF⁻, PI⁻ as an index, live lactic acid bacteria with carbohydrate metabolism activity can be accurately detected.

Example 3

Correlation between Cell Count of Lactic Acid Bacteria with Carbohydrate Metabolism Activity and Colony-Forming Ability:

LcS that has carbohydrate metabolism activity and maintains the cell membrane (hereinafter sometimes referred to as LcS with carbohydrate metabolism activity) contained in commercially available New Yakult, LcS that maintains the cell membrane (hereinafter sometimes referred to as cell membrane-maintaining LcS), and LcS that has a colony-forming ability (hereinafter sometimes referred to as colony-forming LcS) were measured. New Yakult was diluted $2 \times 10^3$ times with DPBS filtered through a 0.22 um filter (hereinafter simply referred to as DPBS), and 200 $\mu$L thereof was dispensed into a 1.5 mL tube. This operation was repeated three times, whereby three tubes in which diluted New Yakult was dispensed were obtained. To each of the tubes, 10 $\mu$L of an L8 antibody (LcS specific antibody) diluted 10-fold with DPBS was added, and the resultant was left to stand at room temperature for 15 minutes, and subsequently, 10 $\mu$L of a 25-fold diluted solution of a DyLight 650-labeled anti-mouse IgM antibody (Thermo Fisher Scientific, # SA5-10153) with DPBS was added thereto, and the resultant was left to stand at room temperature for 15 minutes, thereby preparing an analyte.

Thereafter, in the quantification of the LcS with carbohydrate metabolism activity, 4 $\mu$L of a DMSO solution containing 5 mM carboxyfluorescein diacetate (cFDA) was added to the analyte, and the resultant was left to stand at 30°C for 15 minutes, and then, 8 μL of a 1.5 mM propidium iodide (PI) solution and 8 μL of a 500 mM glucose solution were added thereto, and the resultant was left to stand at 30°C for 15 minutes. Finally, 200 μL of a 10-fold diluted solution of beads with a known concentration, MP-Count Beads (BCT, #7804) with DPBS was added to each bacterial solution and thoroughly mixed with a vortex, and an analysis was performed by Gallios (Beckman Coulter). The concentration of the used beads was $2.15 \times 10^5$ beads/mL. The number of particles to be analyzed was set to 10,000. L8$^+$, cF$^-$, and PI$^-$ were counted as LcS with carbohydrate metabolism activity, and the cell count (cells/mL) was calculated according to the following formula (1).

[Math 1]

$$\text{Cell count (cells/mL)} = \text{Concentration of beads used in test}$$

$$\text{(beads/mL)} \times \text{Count of "bacteria whose cell count is desired to}$$

$$\text{be measured" / Count of "beads"} \times \text{dilution ratio of sample}$$

$$(1)$$

In the quantification of the cell membrane-maintaining LcS, 4 μL of a SYTOX orange solution diluted to 10 μM with Milli-Q water was added to the analyte, and the resultant was left to stand at room temperature for 5 minutes. Thereafter, addition of beads and measurement by Gallios (Beckman Coulter) were performed by the same operation as in the quantification of the LcS with carbohydrate metabolism activity, and L8$^+$ and SYTOX- were counted as cell membrane-maintaining LcS, and the cell count was calculated according to the formula 1.

In the quantification of the colony-forming LcS, the analyte was smeared on MRS agar medium using a spiral plater (EDDY JET2, IUL) and cultured at 37°C in aerobic conditions for 48 to 72 hours, and calculation was performed using a colony counter (Color QCOUNT Model 530, manufactured by Chemopharm Sdn Bhd) from the number of colonies formed. The measurement of the LcS with carbohydrate metabolism activity, cell membrane-maintaining LcS, and colony-forming LcS was performed with New Yakult in multiple lots with different elapsed days from the expiration date (from 13 days before the expiration date to 107 days after the expiration date, at intervals of 3 to 15 days). The results are shown in FIG. 3.

From the results, it was found that the LcS with carbohydrate metabolism activity and the colony forming ability correlate with each other. Therefore, it was found that live lactic acid bacteria can be measured in a shorter time by measuring lactic acid bacteria that have carbohydrate metabolism activity and have maintained the cell membrane than by measuring the colony-forming ability.

Reference Example 1

Time Course of cF Accumulation and cF Excretion of Lactic Acid Bacteria with Carbohydrate Metabolism Activity:

A cultured bacterial solution of LcS in MRS medium was washed by centrifugation with Dulbecco's PBS (DPBS: manufactured by Thermo Fisher Scientific, Inc.), thereby removing saccharides.

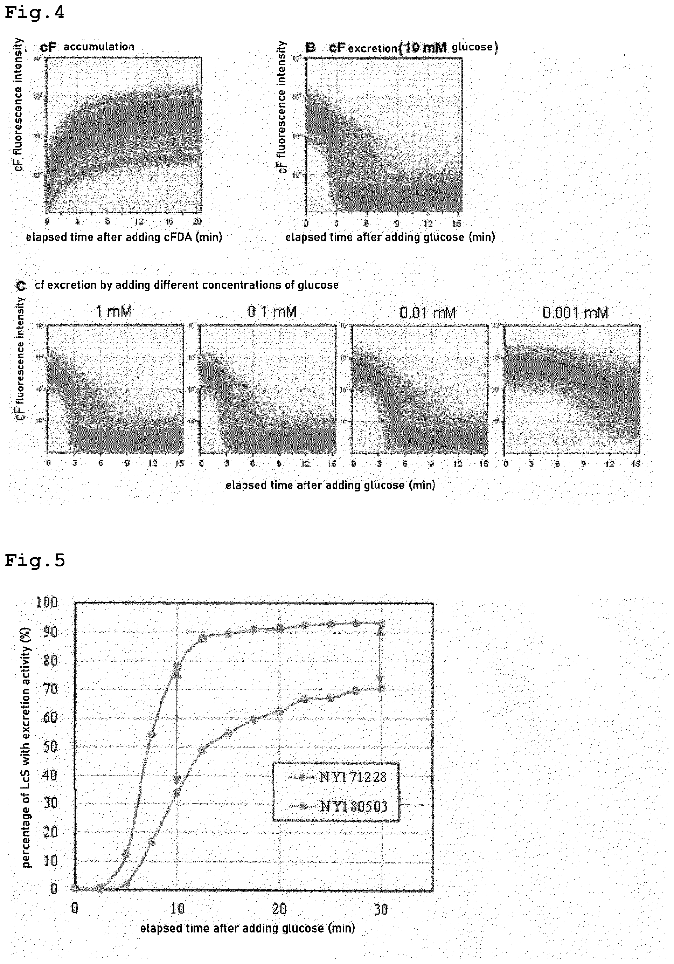

cFDA was added to the DPBS suspension of LcS obtained above at a final concentration of 50 μM (t=0), and cF accumulation from 0 to 20 minutes thereafter was examined by Gallios. The results are shown in FIG. 4A.

In addition, to the DPBS suspension of LcS obtained above, cFDA was added at a final concentration of 50 μM, and the resultant was left to stand at 30°C for 30 minutes to allow cF to accumulate in the bacterial cells. Subsequently, glucose was added at a final concentration of 10 mM (t=0), and cF excretion in 15 minutes thereafter was examined. The results are shown in FIG. 4B.

Further, to the DPBS suspension of LcS obtained above, cFDA was added at a final concentration of 50 μM, and the resultant was left to stand at 30°C for 30 minutes to allow cF to accumulate in the bacterial cells. Subsequently, glucose was added at a final concentration of 0.001 mM, 0.01 mM, 0.1 mM, or 1 mM (t=0), and cF excretion in 15 minutes thereafter was examined. The results are shown in FIG. 4C.

When LcS was treated with cFDA (no sugar was contained at this point), cF rapidly accumulated in the bacterial cells immediately after adding cFDA, and the accumulation amount was almost saturated after 20 minutes (FIG. 4A). Subsequently, when glucose was added at a final concentration of 10 mM to LcS in which cF was accumulated in advance, cF began to be rapidly excreted after about 3 minutes (FIG. 4B). When the final concentration of glucose was decreased to 0.001 mM by 1/10, a slight delay in excretion was observed at 0.01 mM, and a significant delay in excretion was observed at 0.001 mM (FIG. 4C) .

From the above results, it was found that cF is immediately excreted from lactic acid bacteria with carbohydrate metabolism activity by adjusting the hexose having an aldehyde group to 0.01 mM or more. Therefore, it was confirmed that the hexose having an aldehyde group in the lactic acid bacteria-containing beverage or food needs to be adjusted to 0.01 mM or more in order to rapidly measure live lactic acid bacteria with carbohydrate metabolism activity.

Reference Example 2

cF Excretion Activity by Addition of Various Saccharides of LcS:

A beverage or food containing LcS (New Yakult: manufactured by Yakult Honsha Co., Ltd.) stored at about 4°C within 16 days after production was diluted 10-fold with DPBS, then washed by centrifugation three times with DPBS, and the cell pellet was collected.

The cell pellet obtained above was suspended in a 1% (V/W) solution in which a saccharide shown in Table 1 was dissolved in purified water. Further, the suspension was diluted with DPBS so that the cell concentration in the suspension was about $10^6$ cells/mL, and cFDA was added thereto at a final concentration of 50 $\mu$M, and the resultant was left to stand at 30°C for 15 minutes. Subsequently, propidium iodide was added thereto at a final concentration of 30 $\mu$M, and the resultant was left to stand at room temperature for 15 minutes, and then subjected to FACSAria™ fusion (manufactured by Becton Dickinson) to measure the cF fluorescence intensity. A case where 90% or more of the total bacterial cells were counted as cF⁻ was determined to have cF excretion activity. In Table 1, those determined to have cF excretion activity are indicated by +, and those determined to have no cF excretion activity are indicated by . The assimilation of a saccharide was examined using API50cH (manufactured by bioMérieux Japan Co., Ltd.), which is a kit for identifying bacteria. A saccharide that can be assimilated by LcS is indicated by +, and a saccharide that cannot be assimilated by LcS is indicated by -.

[Table 1]

| Saccharide | cF excretion activity | Assimilability | Saccharide | cF excretion activity | Assimilability |
|---|---|---|---|---|---|
| D-glucose | + | + | sucrose | - | + |
| D-galactose | + | + | L-arabinose | - | - |
| D-mannose | + | + | L-rhamnose | - | - |
| L-sorbose | - | + | 2-deoxy-D-glucose | - | unknown |
| Lactose | - | + | 2-deoxy-D-galactose | - | unknown |

From the above results, it was confirmed that cF excretion does not always occur in the presence of an assimilable saccharide, and the presence of glucose, galactose, and mannose, each of which is a hexose having an aldehyde group, is essential for cF excretion.

Reference Example 3

Presence or Absence of cF Excretion Activity of Lactic Acid Bacteria:

A bacterial strain shown in Table 2 precultured in MRS medium was inoculated into 20 mL of another MRS medium at 0.1% (v/v) and cultured at 37°C for 16 to 24 hours, thereby obtaining a cultured bacterial solution. The obtained cultured bacterial solution was washed by centrifugation twice with DPBS, and the cell pellet was collected and suspended in DPBS containing 10 mM glucose. Further, the suspension was diluted with DPBS so that the cell concentration in the suspension was about $10^6$ cells/mL, and cFDA was added thereto at a final concentration of 50 $\mu$M, and the resultant was left to stand at 30°C for 15 minutes. Subsequently, PI was added thereto at a final concentration of 30 $\mu$M, and the resultant was left to stand at room temperature for 15 minutes, and then subjected to FACSAria™ fusion (manufactured by Becton Dickinson) to measure the cF fluorescence intensity. This cF fluorescence intensity was defined as the cF fluorescence intensity in the presence of glucose. Subsequently, the same operation as described above was performed and the cF fluorescence intensity was measured for an analyte obtained by suspending the cell pellet in DPBS in place of DPBS containing 10 mM glucose, and diluting the suspension with DPBS so that the cell concentration was about $10^6$ cells/mL. This cF fluorescence intensity was defined as the cF fluorescence intensity in the absence of glucose. A value obtained by dividing the median value of the cF fluorescence intensity in the presence of glucose by the median value of the cF fluorescence intensity in the absence of glucose was defined as the cF fluorescence intensity ratio. Those with a cF fluorescence intensity ratio of less than 0.1 were determined to be positive for excretion activity (+), and the others were determined to be negative (-). The results are shown in Table 2.

[Table 2]

| Bacterial strain | | Excretion activity |
|---|---|---|
| Lacticaseibacillus paracasei | YIT 0180 | + |
| Lacticaseibacillus paracasei | YIT 9029 | + |
| Lacticaseibacillus paracasei | YIT 10083 | + |
| Lacticaseibacillus paracasei | YIT 11302 | + |
| Lacticaseibacillus paracasei | YIT 11340 | + |
| Lacticaseibacillus casei | YIT 12849 | + |
| Lactobacillus acidophilus | YIT 0070[T] | + |
| Lactobacillus acidophilus | YIT 0191 | + |
| Lactobacillus acidophilus | YIT 0200 | + |
| Lactobacillus acidophilus | YIT 10097 | + |
| Levilactobacillus brevis | YIT 0076[T] | + |
| Lactobacillus gasseri | YIT 0192[T] | + |
| Lactobacillus gasseri | YIT 0285 | + |
| Lactiplantibacillus plantarum | YIT 0102[T] | + |
| Lactiplantibacillus plantarum | YIT 0101 | + |
| Lactiplantibacillus plantarum | YIT 10015 | + |
| Ligilactobacillus salivarius | YIT 0452 | + |
| Lactobacillus helveticus | YIT 0083[T] | + |
| Lactobacillus helveticus | YIT 0131 | + |
| Lactobacillus helveticus | YIT 0464 | + |
| In the table, T denotes a Type strain. | | |

In the results in Table 2, it was confirmed that the detection method of the invention can be applied even when lactic acid bacteria other than LcS are used.

Reference Example 4

Changes in Percentage of LcS with cF Excretion Activity after Addition of Glucose:

New Yakult within the expiration date was diluted 5-fold with DPBS, then 150 μL of a 1 M Tris-HCl [pH 8.0] solution of 38.3 mg/mL protease P Amano 3DS was added thereto and the treatment was performed at 45°C for 3 minutes to remove milk protein. The centrifuged cell pellet was washed once more by centrifugation with DPBS, and the cell pellet was collected with 1 mL of DPBS (10-fold diluted bacterial solution) to remove saccharides. The cell pellet obtained above was further diluted $10^2$ times with DPBS (the cell concentration in the suspension was $10^5$ to $10^6$ cells/mL) .
To the DPBS suspension of LcS obtained above, cFDA was added at a final concentration of 50 μM, and the resultant was left to stand at 30°C for 30 minutes to allow cF to accumulate in the bacterial cells. Subsequently, glucose was added thereto at a final concentration of 10 mM (t=0) and cF excretion was measured over time by Gallios from 0 to 30 minutes thereafter. This operation was performed on two lots. The results are shown in FIG. 5.
From the results, it was found that there is a difference in the percentage of bacteria with cF excretion activity between lots when the measurement is performed within a short time, preferably within 10 minutes after adding glucose to the analyte, and it was confirmed that bacteria with high cF excretion activity can be selected.

Reference Example 5

Measurement Method for Live Cell Count by FCM Using Beads:

(1) Analyzed Bacterial Strain

As the bacterial cells, LcS cultured in MRS liquid medium (hereinafter referred to as "MRS") was used.

(2) Diluent

In the dilution of the bacterial solution and beads, DPBS filtered through a 0.22 um filter was used (hereinafter referred to as "DPBS").

(3) Beads for FCM Counting

As beads with a known concentration for FCM counting, MP-Count Beads (BCT, #7804) were used. The beads have been fluorescently labeled with FL2/PE and have an average particle diameter of 3 um. The bead concentration differs for each product lot, and the concentration of the lot used this time was $2.15 \times 10^6$ beads/mL.

(4) Reagent for Distinguishing between Live and Dead

In the distinction between live and dead bacteria by FCM, 5 mM SYTOX orange (Thermo Fisher Scientific), which is a cell membrane impermeable reagent, was used, and a solution diluted to 10 $\mu$M with Milli-Q was stored at -20°C until use.

(5) Sample Preparation

100 $\mu$L of the bacterial solution after culturing was diluted 10-fold by being mixed with 900 $\mu$L of DPBS, and 10-fold dilution was further repeated twice, thereby preparing a $10^3$-fold diluted solution. 200 $\mu$L of this $10^3$-fold diluted solution was transferred to a new tube, 2 $\mu$L of 10 $\mu$M SYTOX orange was added thereto, and the resultant was left to stand at room temperature for 5 minutes. During this time, a 10-fold diluted solution of the beads for FCM counting with DPBS was prepared. 200 $\mu$L of the 10-fold diluted bead solution ($2.15 \times 10^5$ beads/mL) was added to each sample and thoroughly mixed with a vortex, and then, the entire amount was transferred to an analysis tube and analyzed by FCM. After examining the mixing ratio of the bacterial cells and the beads in (7) described below, the dilution ratio of the bacterial solution to be mixed with the 10-fold diluted bead solution was set to $2 \times 10^3$ times.

(6) FCM Analysis

An analysis was performed by Gallios (Beckman Coulter). Discriminator, Voltage, and Gain setting values for forward scattered light (FS) were 4, 450, and 1, respectively, for side scattered light (SS) were off, 450, 5, respectively, and for FL2 for SYTOX orange detection were off, 470, and 1, respectively. The number of particles to be analyzed per sample was set to 10,000 or 20,000. On the double plot of FL2 and SS, both the FL2 and SS signal intensities of the beads are higher than those of the bacterial cells, so that the region was gated in a "bead" gate. In addition, a population with a lower SS intensity than the beads and a lower FL2 intensity because it was not stained with SYTOX orange was gated in a "live cell" gate. Based on the count of each gate, the live cell count in the sample was calculated using the above formula (1).

(7) Examination of Optimum Mixing Ratio of Bacterial Cells and Beads

In order to examine the range of an appropriate mixing ratio of a sample and beads, $10^3$-fold diluted bacterial solution was used as a reference, and further, 2-fold serial dilutions (2-fold, 4-fold 8-fold, ..., and 512-fold) were prepared, and each of these was mixed with the 10-fold diluted beads. Note that the cultured bacterial solution in one tube was used, which was serially diluted two-fold in three series, and each sample was analyzed once by FCM.

<Results>

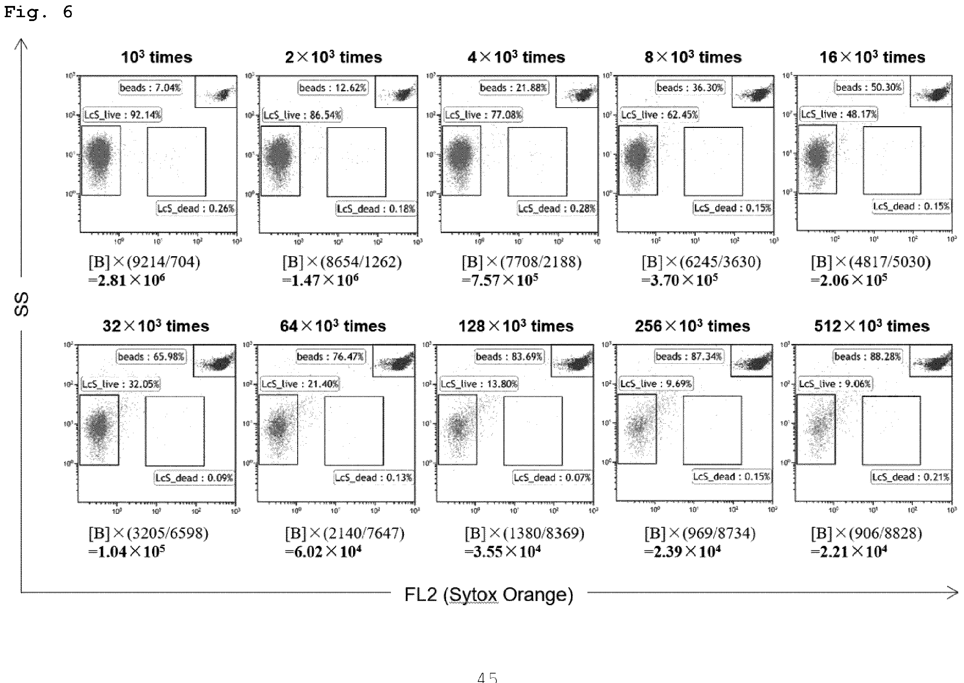

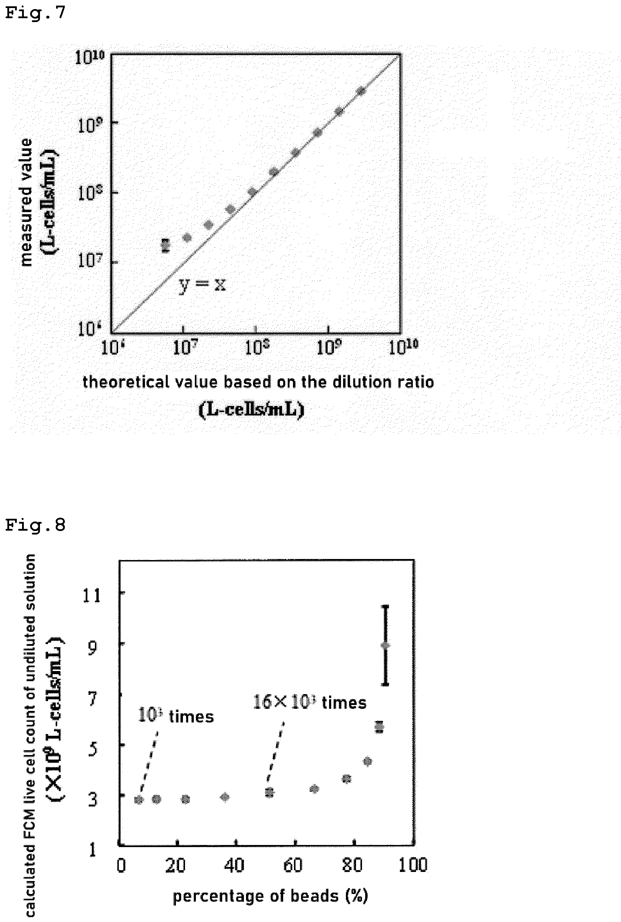

Since the FCM live cell count is calculated from the ratio of the cell count and the bead count, the range of the optimum mixing ratio of the bacterial cells and the beads was first examined. LcS cultured overnight in MRS was used as the bacterial strain, and each of the 2-fold serial dilutions from $10^3$-fold $10^3$-fold, $2 \times 10^3$-fold, $4 \times 10^3$-fold, ..., and $512 \times 10^3$-fold) was mixed with an equal amount of the 10-fold diluted beads ($2.15 \times 10^5$ beads/mL) and an analysis was performed by FCM (n=3). A representative single FL2-SS plot is shown in FIG. 6. In the case of the $10^3$-fold diluted bacterial solution, the bead count was 704, the LcS live cell count was 9214, and the FCM live cell count of the same diluted solution was calculated to be $2.81 \times 10^6$ cells/mL. Similarly, the FCM live cell count was calculated also for the 2-fold serial dilutions and compared with the theoretical value based on the dilution ratio (FIG. 7). With 2-fold dilution, the cell count initially decreased according to the theoretical value, but as the dilution progressed, the measured value showed a higher value than the theoretical value. The reason for this is thought to be that the total number of particles flowing through the FCM decreases by the decrement of the cell count, while the number of beads is constant in the highly diluted sample, resulting in an increase in the effect of noise. Therefore, it was found that if the cell concentration is too low with respect to the beads, the cell count is calculated to be high due to the effect of noise.

Since the operation is easier when the addition amount of beads is constant without changing for each sample, it is desirable to adjust the dilution ratio of the bacterial solution so that the ratio to the beads is within an appropriate range. Therefore, the concentration of the undiluted solution was calculated by multiplying the bacterial solution when measurement was performed at each dilution ratio by the dilution ratio for each case, and the ratio of the beads when diluted at each dilution ratio was plotted on the horizontal axis, and the calculated concentration of the undiluted solution was plotted on the vertical axis (FIG. 8). As a result, it was considered desirable to perform the analysis when the ratio of the beads in the total particles is in the range of 5 to 70%. From the results, it was found that the live cell count of lactic acid bacteria can be measured in the detection method of the invention by using the beads.

Example 4

Prediction of Acid Resistance of Analyte:

(1) Selective Detection of Bacteria with High Excretion Activity:

A beverage or food containing LcS (New Yakult: manufactured by Yakult Honsha Co., Ltd.) stored at 4°C was frozen at -80°C 14 days or 13 days before the expiration date and on the expiration date. Thereafter, the beverage or food was thawed and diluted 5-fold with DPBS, and then, 150 μL of a 1 M Tris-HCl [pH 8.0] solution of 38.3 mg/mL protease P Amano 3DS was added thereto and the treatment was performed at 45°C for 3 minutes to remove milk protein. The centrifuged cell pellet was washed once more by centrifugation with DPBS, and the cell pellet was collected with 1 mL of DPBS (10-fold diluted bacterial solution) to remove saccharides. The cell pellet obtained above was further diluted $10^2$ times with DPBS (the cell concentration in the suspension was $10^5$ to $10^6$ cells/mL), and cFDA was added thereto at a final concentration of 50 μM, and the resultant was left to stand at 30°C for 15 minutes. Subsequently, PI was added thereto at a final concentration of 30 μM, and the resultant was left to stand at room temperature for 15 minutes. Thereafter, glucose was added thereto at 10 mM, and after 10 minutes, an analysis was performed using CytoFlex S (Beckman Coulter). The number of particles to be analyzed was set to 10,000. cF⁻ and PI⁻ were counted as LcS with high carbohydrate metabolism activity, and the percentage of the LcS with high carbohydrate metabolism activity in all lactic acid bacteria was determined. The same operation was performed on 23 types of lots.

(2) Measurement of Acid Resistance:

A beverage or food containing LcS (New Yakult: manufactured by Yakult Honsha Co., Ltd.) stored at 4°C was frozen at -80°C on the expiration date. Thereafter, the beverage or food was thawed, and 5 μL of the beverage or food was added to 225 μL of DPBS, and the resultant was left to stand at 37°C for 1 hour. Thereafter, the obtained sample was diluted 100-fold with DPBS, and to 200 μL of the sample after dilution, 10 μL of an L8 antibody (LcS specific antibody) was added, and the resultant was left to stand at room temperature for 10 minutes, and subsequently, 10 μL of a 25-fold diluted solution of a DyLight 650-labeled anti-mouse IgM antibody (Thermo Fisher Scientific, # SA5-10153) with DPBS was added thereto, and the resultant was left to stand at room temperature for 10 minutes, thereby preparing an analyte. Thereafter, in the quantification of LcS with carbohydrate metabolism activity, 2 μL of a DMSO solution containing 5 mM carboxyfluorescein diacetate (cFDA) was added to the analyte (the concentration in the analyte was 50 μM), and the resultant was left to stand at 30°C for 15 minutes, and then, 4 μL of a 1.5 mM propidium iodide (PI) solution was added thereto (the concentration in the analyte was about 30 μM), and the resultant was left to stand at 30°C for 15 minutes. An analysis was performed using CytoFlex S (Beckman Coulter). The analysis speed was set high, and the number of particles to be analyzed was set to 10,000. L8$^+$, cF$^-$, and PI$^-$ were counted as LcS with carbohydrate metabolism activity (untreated). Further, a similar experiment was performed also for a sample that was left to stand at 37°C for 1 hour using artificial gastric acid adjusted to pH 3.0 in place of DPBS, and LcS with carbohydrate metabolism activity was counted (acid treatment). The above operation was performed in the same manner on the same 23 types of lots as used for the selection of bacteria with high excretion activity. The viability rate during the acid treatment was calculated by dividing the cell count of LcS with carbohydrate metabolism activity during the acid treatment in the same lot by the cell count of untreated LcS with carbohydrate metabolism activity (the results are not shown).

As a result, it was confirmed that in the sample in which the percentage of LcS with high carbohydrate metabolism activity is high, the viability during the acid treatment is also high (having acid resistance).

Example 5

Prediction of Viability After Low-Temperature Storage:

(1) Selective Detection of LcS with High Carbohydrate Metabolism Activity

A beverage or food containing LcS (New Yakult: manufactured by Yakult Honsha Co., Ltd.) was frozen at -80°C immediately after obtaining (14 days or 13 days before the expiration date) or after being stored at 4°C until the expiration date. Thereafter, the beverage or food was thawed and diluted 5-fold with DPBS, and then, 150 μL of a 1 M Tris-HCl [pH 8.0] solution of 38.3 mg/mL protease P Amano 3DS was added thereto and the treatment was performed at 45°C for 3 minutes to remove milk protein. The centrifuged cell pellet was washed once more by centrifugation with DPBS, and the cell pellet was collected with 1 mL of DPBS (10-fold diluted bacterial solution) to remove saccharides. The cell pellet obtained above was further diluted 10$^2$ times with DPBS (the cell concentration in the suspension was 10$^5$ to 10$^6$ cells/mL), and cFDA was added thereto at a final concentration of 50 μM, and the resultant was left to stand at 30°C for 15 minutes. Subsequently, PI was added thereto at a final concentration of 30 μM, and the resultant was left to stand at room temperature for 15 minutes. Thereafter, glucose was added thereto to 10 mM, and after 10 minutes, an analysis was performed using CytoFlex S (Beckman Coulter). The number of particles to be analyzed was set to 10,000. cF$^-$ and PI$^-$ were counted as LcS with high carbohydrate metabolism activity, and the percentage of the LcS with high carbohydrate metabolism activity in all lactic acid bacteria was determined. The same operation was performed on 21 types of lots (the results are not shown).

(2) Confirmation of CFU After Low-Temperature Storage

A beverage or food containing LcS (New Yakult: manufactured by Yakult Honsha Co., Ltd.) was frozen at -80°C immediately after obtaining or after being stored at 4°C until the expiration date. Thereafter, the beverage or food was thawed and diluted with DPBS, and then, smeared on an MRS plate using a spiral plater. The bacteria were cultured at 37°C under anaerobic conditions for 48 hours and counted using a colony counter. The same operation was performed on the same 21 types of lots as in the above (1) (results are not shown).

It was confirmed that in the sample in which the percentage of LcS with high carbohydrate metabolism activity is high immediately after production, CFU is maintained at a high level after low-temperature storage. Although the correlation between high carbohydrate metabolism activity and CFU rank was low before storage, a correlation was observed after storage. From this, it was confirmed that the viability of the analyte after low-temperature storage can be predicted by measuring the percentage of LcS with high carbohydrate metabolism activity.

Example 6

Correlation between Excretion Score and Percentage of LcS with High Carbohydrate Metabolism Activity:

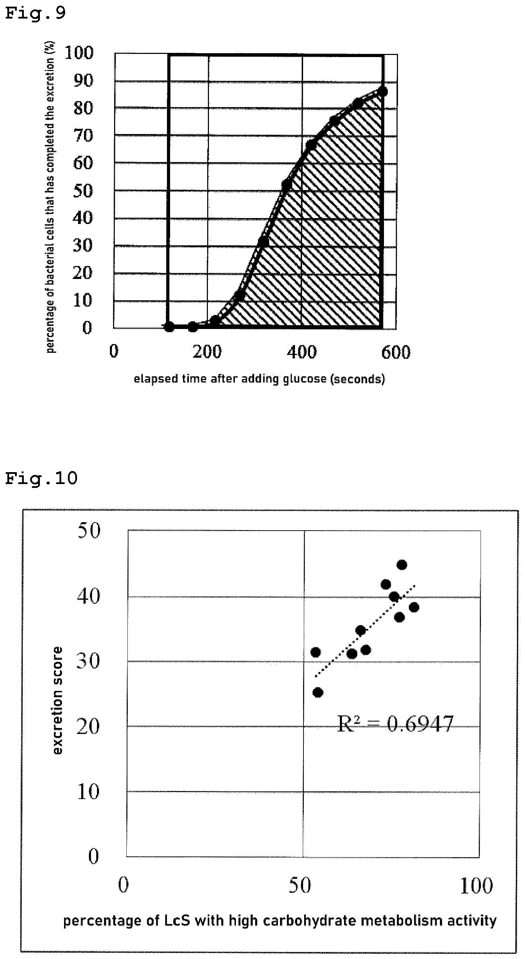

A beverage or food containing LcS (New Yakult: manufactured by Yakult Honsha Co., Ltd.) was used, and the percentage (%) of LcS with high carbohydrate metabolism activity was measured in the same manner as in Example 4. Further, for a product of the same lot, a cell pellet was prepared in the same manner as in Example 4, and the obtained cell pellet was further diluted 10$^2$ times with DPBS (the cell concentration in the suspension was 10$^5$ to 10$^6$ cells/mL), and cFDA was added thereto at a final concentration of 50 μM, and the resultant was left to stand at 30°C for 15 minutes. Subsequently, PI was added thereto at a final concentration of 30 μM, and the resultant was left to stand at room temperature for 15 minutes. Thereafter, glucose was added thereto to 10 mM, and an analysis was performed continuously for 8 minutes from 2 minutes to 10 minutes after the addition using CytoFlex S (Beckman Coulter). From the 8-minute data obtained, 25-second data at 10 measurement points (120, 170, 220, 320, 370, 420, 470, 520, and 570 seconds after adding glucose) were extracted, and the percentage of LcS (cF⁻, PI⁻) that have no cell membrane damage and have completed the excretion of the fluorescent substance was calculated at each measurement point. The percentage was plotted on the vertical axis, and the elapsed time after adjusting glucose to 0.01 mM or more was plotted on the horizontal axis (FIG. 9). By performing trapezoidal approximation of areas between plots and adding up the areas, the area of the curve from 2 minutes to 10 minutes after adding glucose was calculated (Table 3). Subsequently, a percentage calculated by dividing by the area (45000) of a rectangle assuming that the percentage of LcS that has completed the excretion in the corresponding time (from 120 seconds to 570 seconds thereafter) is always 100% was defined as the excretion score (18286/45000 × 100 = 40.6).

[Table 3]

| Time [s] | cF-PI- [%] | Area |
|---|---|---|
| 120 | 0.3 | 19 |
| 170 | 0.5 | 78 |
| 220 | 2.7 | 367 |
| 270 | 12.0 | 1095 |
| 320 | 31.8 | 2095 |
| 370 | 52.0 | 2960 |
| 420 | 66.4 | 3543 |
| 470 | 75.3 | 3930 |
| 520 | 81.9 | 4200 |
| 570 | 86.1 | |
| | | 18286 |

In addition, for samples of 10 lots in total, the percentage (%) of LcS with high carbohydrate metabolism activity and the excretion score were calculated in the same manner as in Example 6, and the correlation is shown in FIG. 10.

As a result, $R^2$ = 0.697947, and it was found that a correlation is observed between the excretion score and the percentage of LcS with high carbohydrate metabolism activity. Therefore, it was confirmed that even the method using the excretion score can selectively detect LcS with high carbohydrate metabolism activity.

Industrial Applicability

According to the invention, live lactic acid bacteria with carbohydrate metabolism activity can be rapidly measured, and the invention can be used for the development of a lactic acid bacteria-containing beverage or food, the quality control thereof, and the like.

**Claims**

1. A method for detecting live lactic acid bacteria with carbohydrate metabolism activity contained in a lactic acid bacteria-containing beverage or food, **characterized by** including the following steps (a) to (c):

   (a) a step of adding a reagent that exhibits fluorescence when degraded by an esterase and a reagent for determining cell membrane damage to a lactic acid bacteria-containing beverage or food;
   (b) a step of adjusting a hexose having an aldehyde group contained in the lactic acid bacteria-containing beverage or food to 0.01 mM or more; and
   (c) a step of detecting lactic acid bacteria that excrete a fluorescent substance and have no cell membrane damage from the lactic acid bacteria-containing beverage or food.

2. The detection method according to claim 1, wherein the step (c) is performed within 60 minutes after the step (b).

3. The detection method according to claim 1 or 2, wherein the reagent that exhibits fluorescence when degraded by an esterase used in the step (a) is carboxyfluorescein diacetate, and the reagent for determining cell membrane damage is propidium iodide or Sytox.

4. The detection method according to any one of claims 1 to 3, wherein the lactic acid bacteria-containing beverage or food contains a milk component.

5. The detection method according to any one of claims 1 to 4, wherein the hexose having an aldehyde group used in the step (b) is one type or two or more types selected from the group consisting of glucose, galactose, and mannose.

6. The detection method according to any one of claims 1 to 5, wherein the lactic acid bacteria are lactic acid bacteria belonging to the Lactobacillus family or lactic acid bacteria belonging to the Streptococcus family.

7. The detection method according to any one of claims 1 to 5, wherein the lactic acid bacteria are lactic acid bacteria belonging to any of the genus Lacticaseibacillus, the genus Lactobacillus, the genus Lactiplantibacillus, the genus Levilactobacillus, the genus Ligilactobacillus, and the genus Lactococcus.

8. The detection method according to any one of claims 1 to 5, wherein the lactic acid bacteria are one strain or two or more strains selected from the group consisting of Lacticaseibacillus paracasei, Lacticaseibacillus casei, Lactobacillus acidophilus, Lactobacillus gasseri, Lactobacillus helveticus, Lactiplantibacillus plantarum, Levilactobacillus brevis, Ligilactobacillus salivarius, and Lactococcus lactis.

9. The detection method according to any one of claims 1 to 8, further comprising adding beads for concentration measurement to the lactic acid bacteria-containing beverage or food, and measuring the live cell count of lactic acid bacteria.

10. The detection method according to claim 9, wherein the concentration of the beads is set to 5 to 70% of the particles contained in the lactic acid bacteria-containing beverage or food.

11. A method for selectively detecting live lactic acid bacteria with high carbohydrate metabolism activity, **characterized by** including the following steps (d) to (f):

(d) a step of adding a reagent that exhibits fluorescence when degraded by an esterase and a reagent for determining cell membrane damage to an analyte containing lactic acid bacteria;
(e) a step of adjusting a hexose having an aldehyde group in the analyte to 0.01 mM or more; and
(f) a step of detecting lactic acid bacteria that excrete a fluorescent substance and have no cell membrane damage from the analyte within 10 minutes after the step (e).

12. The method for selectively detecting lactic acid bacteria with high carbohydrate metabolism activity according to claim 11, wherein the analyte containing lactic acid bacteria used in the step (d) is one from which a saccharide has been removed.

13. The selective detection method according to claim 11 or 12, wherein the reagent that exhibits fluorescence when degraded by an esterase used in the step (d) is carboxyfluorescein diacetate, and the reagent for determining cell membrane damage is propidium iodide or Sytox.

14. The selective detection method according to any one of claims 11 to 13, wherein in (f), a percentage of lactic acid bacteria that have no cell membrane damage and have completed the excretion of a fluorescent substance is plotted on a vertical axis, and an elapsed time after adjusting the hexose having an aldehyde group to 0.01 mM or more is plotted on the horizontal axis, and the detection of lactic acid bacteria that have no excretion of the fluorescent substance nor cell membrane damage is performed based on an area value of the plotted curve.

15. A method for predicting acid resistance of lactic acid bacteria contained in an analyte, **characterized by** using the selective detection method according to any one of claims 11 to 14.

16. A method for predicting viability after low-temperature storage of lactic acid bacteria contained in an analyte, **characterized by** using the selective detection method according to any one of claims 11 to 14.

EP 4 350 000 A1

Fig.1

Fig.2

Fig.3

17

Fig.4

A cF accumulation

B cF excretion(10 mM glucose)

C cf excretion by adding different concentrations of glucose

1 mM 0.1 mM 0.01 mM 0.001 mM

elapsed time after adding glucose (min)

Fig.5

NY171228
NY180503

elapsed time after adding glucose (min)

Fig. 6

Fig. 6

EP 4 350 000 A1

Fig.7

Fig.8

Fig.9

Fig.10

## INTERNATIONAL SEARCH REPORT

International application No.

**PCT/JP2022/020322**

| A. | CLASSIFICATION OF SUBJECT MATTER |
|---|---|

*C12Q 1/04*(2006.01)i; *C12N 1/20*(2006.01)i; *G01N 33/48*(2006.01)i
FI:    C12Q1/04; C12N1/20 F; G01N33/48 M

According to International Patent Classification (IPC) or to both national classification and IPC

| B. | FIELDS SEARCHED |
|---|---|

Minimum documentation searched (classification system followed by classification symbols)

C12Q1/04; C12N1/20; G01N33/48

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | SUNNY-ROBERTS, E. O. et al. Evaluation of the response of Lactobacillus rhamnosus VTT E-97800 to sucrose-induced osmotic stress. Food Microbiology. 02 June 2007, vol. 25, issue 1, pp. 183-189<br>13. Extrusion of intracellular accumulated dye, fig. 3, 4 | 1-16 |
| A | BUNTHOF, C. J. et al. Rapid fluorescence assessment of the viability of stressed Lactococcus lactis. Applied and Environmental Microbiology. 01 August 1999, vol. 65, no. 8, pp. 3681-3689<br>abstract, fig. 6, table 1 | 1-16 |
| A | BUNTHOF, C. J. et al. Fluorescence assessment of Lactococcus lactis viability. International Journal of Food Microbiology. 10 April 2000, vol. 55, issues 1-3, pp. 291-294<br>abstract, fig. 1-3 | 1-16 |

☐ Further documents are listed in the continuation of Box C.     ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 June 2022** | **05 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H04218392 A

**Non-patent literature cited in the description**

- **LAHTINEN SJ ; OUWEHAND AC ; REINIKAINEN JP ; KORPELA JM ; SANDHOLM J ; SALMINEN SJ.** Intrinsic properties of so-called dormant probiotic bacteria, determined by flow cytometric viability assays. *Appl Environ Microbiol,* 2006, vol. 72, 5132-4

- **ZHENG et al.** *A taxonomic note on the genus Lactobacillus: Description of 23 novel genera, emended description of the genus Lactobacillus Beijerinck,* 1901
- *Int. J. Syst. Evol. Microbiol.,* April 2020, vol. 70 (4), 2782-2858